# EUROPÄISCHE PATENTSCHRIFT

(11) **EP 0 859 578 B1**
(45) Veröffentlichungstag und Bekanntmachung des Hinweises auf die Patenterteilung: **08.08.2001**
(21) Anmeldenummer: 96934307.8
(22) Anmeldetag: 08.11.1996
(51) Int. Cl.: A61F 2/34

(54) **KÜNSTLICHE HÜFTGELENKPFANNE**
ARTIFICIAL COTYLOID CAVITY
CAVITE COTYLOIDE ARTIFICIELLE

(30) Priorität: 08.11.1995 CH 316195
(43) Veröffentlichungstag der Anmeldung: 26.08.1998
(73) Patentinhaber: Stratec Medical AG, 4436 Oberdorf (CH)
(72) Erfinder: SUEMER, Aykut, 80620 Istanbul (TR)
(74) Vertreter: Lusuardi, Werther Giovanni, Dr.
(86) Internationale Anmeldenummer: CH9600395
(87) Internationale Veröffentlichungsnummer: WO9717040

(56) Entgegenhaltungen:
- EP-A- 0 083 708
- EP-A- 0 237 751
- EP-A- 0 242 633
- EP-A- 0 444 382
- EP-A- 0 578 322
- EP-A- 0 655 230
- WO-A-95/01139
- WO-A-95/22944
- US-A- 3 584 318
- US-A- 3 863 273

## Beschreibung

Die Erfindung betrifft eine künstliche Hüftgelenkpfanne, gemäss dem Oberbegriff des Patentanspruchs 1.

Solche sphärische Hüftgelenkpfannen eignen sich insbesondere für die zementfreie Implantation in das Acetabulum.

Aus dem Stand der Technik (EP-A 444 382) ist eine derartige Hüftgelenkpfanne als "Monoblock" bekannt. Die Nachteile dieser Anordnung bestehen darin, dass die Aussenschale eine konstante Wandstärke und eine Sandwich-Bauweise aufweist. Dadurch wird die Elastizität eingeschränkt. Die Aufhängung zwischen Aussen- und Innenschale, muss durch ein zusätzlich angebrachtes Federungssystem geregelt werden und ist daher nicht selbsttragend.

Aus der EP-A-0655230 ist eine künstliche Hüftgelenkpfanne bekannt mit einer aussen konischen Innenschale und einer innen konischen Aussenschale, welche einen Hohlraum zwischen sich bilden und an ihren Rändern durch einen separaten Haltering und Schrauben gegeneinander fixierbar sind. Die Wandstärke der Aussenschale verjüngt sich anfänglich in Richtung des Randes, verdickt sich dann in einem mittleren Teil, um sich schliesslich wieder gegen den Rand hin zu verjüngen. Auch bei dieser bekannten Hüftgelenkpfanne ist somit die Elastizität eingeschränkt.

Hier will die Erfindung Abhilfe schaffen. Der Erfindung liegt die Aufgabe zugrunde, eine künstliche Hüftgelenkpfanne zu schaffen, welche in Richtung zum Pol hin zunehmend elastisch ausgebildet ist und trotzdem eine einfache und kostengünstige Konstruktion aufweist.

Zur Lösung dieses Problemes ist die eingangs genannte Anordnung durch die Merkmale des kennzeichnenden Teils des unabhängigen Anspruchs 1 weitergebildet.

Damit ist der Vorteil erzielbar, dass in Richtung der resultierenden Belastungsachse genügend mechanische Stabilität, jedoch im Bereich des Poles der künstlichen Hüftgelenkpfanne, d.h. dort wo das Implantat im ausgefrästen Acetabulum seinen tiefsten Punkt erreicht, eine optimale Elastizität und Adaptabilität vorhanden ist.

Die Wandstärke der Aussenschale ist an ihrem Pol geringer als an ihrem 20. - 40. Breitengrad (vorzugsweise ihrem 25. - 35. Breitengrad), dem ungefähren Ort des Beginns des erfindungsgemässen Elastizitätsbereiches, und beträgt am Pol weniger als 0,30 mm, vorzugsweise weniger als 0,25 mm. Am 30. Breitengrad beträgt die Wandstärke zweckmässigerweise etwa 0,5 mm. Die Wandstärke der Aussenschale sollte an ihrem Pol 30 - 60 % der Wandstärke der Aussenschale an ihrem 30. Breitengrad betragen.
Der Rand der Aussenschale besitzt eine zur Verankerung im Knochen bestimmte Form und Aussenfläche, die kegelförmige Randform ermöglicht ein primäres Verklemmen - in der Fachsprache "pressfit" genannt - zur Verhinderung von Kippbewegungen. Die Rotationssicherung wird durch axial verlaufende Randnuten erreicht. Die Mantelfläche des Randes weist eine dreidimensionale Strukturierung auf. Diese Strukturierung hat eine Tiefe von 0,3 bis 0,8 mm, vorzugsweise von 0,4 - 0,6 mm um das An-, bzw. Einwachsen des Knochens zu erleichtern.
Die Aussenschale kann aus Reintitan oder einer Titanlegierung gefertigt werden, während die Innenschale vorteilhafterweise aus einem der folgenden Materialien besteht: Kobalt-Chrom-Molybdän-Legierung, Keramik, Polyethylen oder kohlenfaserverstärkter Kunststoffe.

Fabrikatorisch kann ein ganzes Set mit mehreren Hüftgelenkpfannen verschiedener Grösse hergestellt werden, wobei für sämtliche Hüftgelenkpfannen identische Innenschalen verwendet werden und die verschiedenen Grössen durch verschieden grosse Aussenschalen realisiert werden.

Die durch die Erfindung erreichten Vorteile sind im wesentlichen darin zu sehen, dass dank der erfindungsgemässen Hüftgelenkpfanne:
1. Eine den physiologischen Elastizität-Begebenheiten des Acetabulums adaptierte Lösung in Verankerung und Funktionalität gegeben ist;
2. ein fertigungstechnisch einfacher Baukasten mit einem Minimum an zusätzlichem Instrumentarium besteht; und dadurch
3. eine denkbar einfache und problemminimierende Operationstechnik angewandt werden kann.
Die Erfindung und Weiterbildungen der Erfindung werden im folgenden anhand der teilweise schematischen Darstellungen mehrerer Ausführungsbeispiele noch näher erläutert.
Es zeigen:
Fig. 1 einen vergrösserten Querschnitt durch die Drehachse der erfindungsgemässen Hüftgelenkpfanne;
Fig. 2 eine Ansicht in natürlicher Grösse von unten in Pfeilrichtung A in das Innere der erfindungsgemässen Hüftgelenkpfanne nach Fig. 1; und
Fig. 3 einen stark vergösserten Detailquerschnitt im Bereich des Aussenrandes der Hüftgelenkpfanne nach Fig. 1.

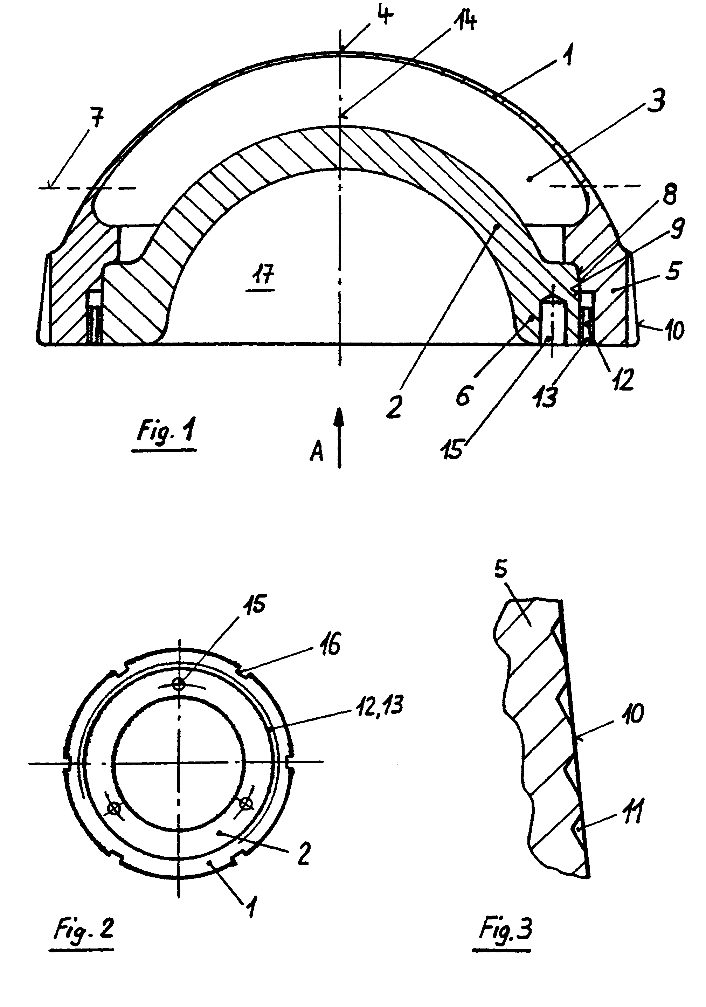

Die in den Fig. 1 und 2 dargestellte künstliche Hüftgelenkpfanne besteht einerseits aus einer kalottenförmigen Aussenschale 1 mit verstärktem Rand 5 und Innengewinde 12 und anderseits aus einer kalottenförmigen Innenschale 2 mit Rand 6 und Aussengewinde 13, welche einen halbkugelförmigen Innenraum 17 zur Aufnahme des zeichnerisch nicht dargestellten Kopfes des Femurteils einer Hüftprothese bildet. Bei der Herstellung der künstliche Hüftgelenkpfanne werden die beiden Schalen 1,2 mittels ihrer Gewinde 12,13 miteinander verschraubt.
Der Rand 6 der Innenschale 2 weist im weiteren eine kegelförmige Aussenfläche 8 und der Rand 5 der Aussenschale 1 eine kegelförmige Innenfläche 9 auf, welche anlässlich der fabrikatorischen Verschraubung der beiden Schalen 1,2 reibschlüssig gegeneinander zur Anlage kommen, wie dies in Fig. 1 dargestellt ist. Die Gewinde 12,13 können fabrikatorisch durch Verkrimpung unlöslich gemacht werden, so dass insgesamt die Aussen- und Innenschalen 1,2 an ihren Rändern 5,6 fest miteinander verbunden sind und zwischen sich einen gas- und flüssigkeitsdichten Hohlraum 3 bilden.

Die Wandstärke der Aussenschale 1 beträgt an ihrem Pol 4 lediglich 0,2 mm und wächst bis zu ihrem ungefähren 30. Breitengrad kontinuierlich auf 0,5 mm an.

Die Innenschale 2 ist - wie in den Fig. 1 und 2 gezeigt - an ihrem Rand 6 mit drei Sacklöchern 15 versehen, welche im wesentlichen parallel zur Drehachse 14 der Hüftgelenkpfanne verlaufen. Die Sacklöcher 15 dienen dazu, die Hüftgelenkpfanne mittels eines geeigneten - zeichnerisch nicht dargestellten - darin eingreifenden Instrumentes zu Handhaben. Die konische Aussenfläche 10 ist mit axialen Randnuten 16 umfasst.

Wie in Fig. 3 gezeigt, besitzt der aussen konisch ausgebildete, verstärkte Rand 5 der Aussenschale 1 eine zur Anlage an den Knochen bestimmte Aussenfläche 10, welche eine dreidimensionale Strukturierung 11 mit einer Tiefe von 0,5 mm aufweist.

## Patentansprüche

1. Künstliche Hüftgelenkpfanne mit einer kalottenförmigen Aussenschale (1) und einer kalottenförmigen Innenschale (2), welche einen Hohlraum (3) zwischen sich bilden, wobei
A) die Aussen- und Innenschalen (1,2) an ihren Rändern (5,6) miteinander lagestabil verbunden sind, und
B) die Aussenschale (1) unterhalb eines zwischen dem 20. und 40. Breitengrad liegenden Übergangskreises (7) in einen verstärkten Rand (5) übergeht, **dadurch gekennzeichnet, dass**
C) die Wandstärke der Aussenschale (1) ausgehend von ihrem Pol (4) in Richtung ihres Randes (5) kontinuierlich zunimmt, und
D) die Wandstärke der Aussenschale (1) an ihrem Pol (4) geringer als 0,30 mm ist.

2. Hüftgelenkpfanne nach Anspruch 1, dadurch gekennzeichnet, dass die Wandstärke der Aussenschale (1) ausgehend von ihrem Pol (4) in Richtung des zwischen dem 20. - 40. Breitengrad, vorzugsweise zwischen dem 25. - 35. Breitengrad der Aussenschale (1) liegenden Übergangskreises (7) kontinuierlich zunimmt.

3. Hüftgelenkpfanne nach Anspruch 1 oder 2, dadurch gekennzeichnet, dass die Wandstärke der Aussenschale (1) an ihrem Pol (4) geringer als 0,25 mm ist.

4. Hüftgelenkpfanne nach einem der Ansprüche 1 - 3, dadurch gekennzeichnet, dass die Wandstärke der Aussenschale (1) an ihrem Pol (4) 30 - 60 % der Wandstärke der Aussenschale (1) auf der Höhe des Übergangskreises (7) beträgt.

5. Hüftgelenkpfanne nach einem der Ansprüche 1 bis 4, dadurch gekennzeichnet, dass der Rand (5) der Aussenschale (1) ein Innengewinde (12) und der Rand (6) der Innenschale (2) ein darauf abgestimmtes Aussengewinde (13) zur Verschraubung der beiden Schalen (1,2) aufweisen.

6. Hüftgelenkpfanne nach einem der Ansprüche 1 bis 5, dadurch gekennzeichnet, dass der Rand (6) der Innenschale (2) eine kegelförmige Aussenfläche (8) aufweist und der Rand (5) der Aussenschale (1) eine kegelförmige Innenfläche (9) aufweist, welche mit der Aussenfläche (8) reibschlüssig verbunden ist.

7. Hüftgelenkpfanne nach einem der Ansprüche 1 bis 6, dadurch gekennzeichnet, dass der Rand (5) der Aussenschale (1) eine zur Anlage an den Knochen bestimmte, konische Aussenfläche (10) mit axialen Randnuten (16) umfasst, welche eine dreidimensionale Strukturierung (11) aufweist.

8. Hüftgelenkpfanne nach Anspruch 7, dadurch gekennzeichnet, dass die Strukturierung (11) eine Tiefe von 0,3 bis 0,8 mm, vorzugsweise von 0,4 - 0,6 mm aufweist.

9. Hüftgelenkpfanne nach einem der Ansprüche 1 bis 8, dadurch gekennzeichnet, dass die Aussenschale (1) aus Reintitan oder einer Titanlegierung besteht.

10. Hüftgelenkpfanne nach einem der Ansprüche 1 bis 9, dadurch gekennzeichnet, dass die Innenschale (2) aus einem der folgenden Materialien besteht: Kobalt-Chrom-Molybdän-Legierung, Keramik Polyethylen oder kohlenfaserverstärkter Kunststoffe.

11. Hüftgelenkpfanne nach einem der Ansprüche 1 bis 10, dadurch gekennzeichnet, dass die Innenschale (2) an ihrem Rand (6) mit mindestens 3 Sacklöcher (15) versehen ist, welche im wesentlichen parallel zur Drehachse (14) der Hüftgelenkpfanne verlaufen.

12. Set mit mehreren Hüftgelenkpfannen verschiedener Grösse nach einem der Ansprüche 1 - 11, dadurch gekennzeichnet, dass die einzelnen Hüftgelenkpfannen identische Innenschalen (2) und verschieden grosse Aussenschalen (1) aufweisen.

## Claims

1. Artificial cotyloid socket having a domal outer shell (1) and a domal inner shell (2) subtending therebetween a cavity (3), whereby
A) the outer and inner shells (1;2) being joined to each other positionally stable at their rims (5;6); and
B) the outer shell (1) merging into a reinforced rim (5) beneath a circle of merging (7) lying between the 20^{th} and the 40^{th} degree of latitude,
characterized in that
C) the wall thickness of the outer shell (1) continuously increases as one moves from said pole (4) toward said rim (5), and
D) the wall thickness of the outer shell (1) is less than 0,30 mm at said pole (4).

2. Cotyloid socket according to claim 1, characterized in that the wall thickness of the outer shell (1) continuously increases as one moves from said pole (4) toward said circle of merging (7) lying between the 20^{th} and the 40^{th} preferably between the 25^{th} and 35^{th} degree of latitude of the outer shell (1).

3. Cotyloid socket according to claim 1 or 2, characterized in that the wall thickness of the outer shell (1) is less than 0,25 mm at said pole (4).

4. Cotyloid socket according to one of the claims 1 to 3, characterized in that the wall thickness of the outer shell (1) at said pole (4) is between 30 - 60% of the wall thickness of the outer shell (1) at the height of the circle of merging (7).

5. Cotyloid socket according to one of the claims 1 to 4, characterized in that the rim (5) of the outer shell (1) has an internal thread (12) and that the rim (6) of the inner shell (2) has an external thread (13) matching thereto to permit the two shells (1;2) to be threadably attached to one another.

6. Cotyloid socket according to one of the claims 1 to 5, characterized in that the rim (6) of the inner shell (2) comprises a conical outer surface (8) and the rim (5) of the outer shell (1) comprises a conical inner surface (9) which is connected in a frictionally-locking manner to said outer surface (8).

7. Cotyloid socket according to one of the claims 1 to 6, characterized in that the rim (5) of the outer shell (1) comprises a conical outer surface (10) being adapted to contact a bone and having axial rim grooves (16), which has a three-dimensional structure (11).

8. Cotyloid socket according to claim 7, characterized in that the structure (11) has a depth of 0,3 to 0,8 mm, preferably of 0,4 - 0,6 mm.

9. Cotyloid socket according to one of the claims 1 to 8, characterized in that the outer shell (1) is made of pure titanium or a titanium alloy.

10. Cotyloid socket according to one of the claims 1 to 9, characterized in that the inner shell (2) is made of one of the following materials: cobalt-chromium-molybdenum alloy, ceramics, polyethylene or carbon-fiber reinforced synthetic.

11. Cotyloid socket according to one of the claims 1 to 10, characterized in that the inner shell (2) is provided at said rim (6) with at least three blind holes (15) which extend substantially parallel to the axis of rotation (14) of the cotyloid socket.

12. Set of multiple cotyloid sockets of different size according to one of the claims 1 - 11, characterized in that the individual cotyloid sockets are provided with identical inner shells (2) and with outer shells (1) differently sized.

## Revendications

1. Cavité cotyloïde artificielle présentant une coquille extérieure (1) en forme de calotte et une coquille intérieure (2) en forme de calotte qui forment entre elles un espace creux (3), dans laquelle :
A) la coquille extérieure et la coquille intérieure (1, 2) sont reliées l'une à l'autre en position stable par leurs bords (5, 6) et
B) la coquille extérieure (1) se transforme en un bord renforcé (5) en dessous d'un cercle de transition (7) situé entre le 20ème et le 40ème degré de latitude,
caractérisée en ce que
C) partant de son pôle (4), l'épaisseur de la paroi de la coquille extérieure (1) augmente de manière continue en direction de son bord (5), et
D) en son pôle (4), l'épaisseur de la paroi de la coquille extérieure (1) est inférieure à 0,30 mm.

2. Cavité cotyloïde selon la revendication 1, caractérisée en ce que partant de son pôle (4), l'épaisseur de la paroi de la coquille extérieure (1) augmente de manière continue en direction du cercle de transition (7) situé entre le 20ème et le 40ème degré de latitude, de préférence entre le 25ème et le 35ème degré de latitude de la coquille extérieure (1).

3. Cavité cotyloïde selon la revendication 1 ou 2, caractérisée en ce qu'en son pôle (4), l'épaisseur de la paroi de la coquille extérieure (1) est inférieure à 0,25 mm.

4. Cavité cotyloïde selon l'une des revendications 1 à 3,
caractérisée en ce qu'en son pôle (4), l'épaisseur de la paroi de la coquille extérieure (1) représente de 30 à 60 % de l'épaisseur de la paroi de la coquille extérieure (1) à la hauteur du cercle de transition (7).

5. Cavité cotyloïde selon l'une des revendications 1 à 4,
caractérisée en ce que le bord (5) de la coquille extérieure (1) présente un filet intérieur (12) et en ce que le bord (6) de la coquille intérieure (2) présente un filet extérieur (13) accordé sur le premier, pour le vissage des deux coquilles (1, 2).

6. Cavité cotyloïde selon l'une des revendications 1 à 5,
caractérisée en ce que le bord (6) de la coquille intérieure (2) présente une surface extérieure (8) de forme conique et en ce que le bord (5) de la coquille extérieure (1) présente une surface intérieure (9) de forme conique qui est reliée par frottement à la surface extérieure (8).

7. Cavité cotyloïde selon l'une des revendications 1 à 6,
caractérisée en ce que le bord (5) de la coquille extérieure (1) comporte une surface extérieure (10) conique, dotée de rainures axiales de bord (16) qui présentent une structuration tridimensionnelle (11), et qui est destinée à venir se placer contre l'os.

8. Cavité cotyloïde selon la revendication 7, caractérisée en ce que la structuration (11) présente une profondeur de 0,3 à 0,8 mm, de préférence de 0,4 à 0,6 mm.

9. Cavité cotyloïde selon l'une des revendications 1 à 8,
caractérisée en ce que la coquille extérieure (1) est réalisée en titane pur ou en alliage de titane.

10. Cavité cotyloïde selon l'une des revendications 1 à 9,
caractérisée en ce que la coquille intérieure (2) est constituée de l'un des matériaux suivants : alliage cobalt-chrome-molybdène, céramique, polyéthylène ou matières synthétiques renforcées de fibres de carbone.

11. Cavité cotyloïde selon l'une des revendications 1 à 10,
caractérisée en ce que la coquille intérieure (2) présente sur son bord (6) au moins trois trous aveugles (15) qui s'étendent essentiellement en parallèle à l'axe de rotation (14) de la cavité cotyloïde.

12. Trousse comportant plusieurs cavités cotyloïdes de différentes tailles selon l'une des revendications 1 à 11, caractérisée en ce que les cavités cotyloïdes individuelles présentent des coquilles intérieures (2) identiques et des coquilles extérieures (11) de différentes tailles.
